# EUROPEAN PATENT SPECIFICATION

(11) **EP 2 574 167 B1**
(45) Date of publication and mention of the grant of the patent: **16.12.2015**
(21) Application number: 11726367.3
(22) Date of filing: 20.05.2011
(51) Int. Cl.: A61K 9/00, A61K 31/485, A61K 31/55

(54) **LIQUID NASAL SPRAY CONTAINING LOW-DOSE NALTREXONE**
FLÜSSIGES NASENSPRAY MIT NIEDRIGDOSIERTEM NALTREXON
SPRAY NASAL LIQUIDE CONTENANT DU NALTREXONE À FAIBLE DOSE

(30) Priority: 21.05.2010 IT FI20100113
(43) Date of publication of application: 03.04.2013
(73) Proprietor: L. MOLTENI & C. DEI FRATELLI ALITTI SOCIETA' DI ESERCIZIO S.P.A, 50018 Scandicci (IT)
(72) Inventor: ANGELI, Roberto, I-50018 Scandicci (IT); RAFFAELI, William, I-47923 Rimini (IT); RIGAMONTI, Maria Adele, I-50145 Firenze (IT)
(74) Representative: Brighenti, Livio
(86) International application number: PCT/EP2011/058284
(87) International publication number: WO 2011/144746

(56) References cited:
- WO-A1-00/62757
- WO-A1-2009/040595
- WO-A2-03/002071
- US-A1- 2003 077 300
- US-A1- 2007 212 307

## Description

### FIELD OF THE INVENTION

The present invention relates to the field of compositions for nasal administration of drugs of the opioid antagonist category.

### PRIOR ART

The opioids are drugs that produce an analgesic and antinociceptive response with different degrees of potency depending on the pharmaceutical substance used. There are numerous pharmaceutical substances of the opioid and/or opiate category, such as morphine, fentanyl, hydromorphone, tramadol, codeine, buprenorphine, oxycodone, methadone. All the drugs in this category are characterized, in clinical use, by the capacity to induce an analgesic effect and to generate, in various ways, other effects that are not correlated with the therapeutic expectation, which are grouped under the general term of "adverse effects". The presence of these adverse effects, poorly tolerated by the patient, often leads, depending on their intensity and severity, to discontinuation of treatment with the opioid, even though it is needed for controlling pain.

It is known that all the pharmacological effects of formulations with opioids are due to the pharmacodynamic effect of interaction of the opioids with specific receptors, called "opioid receptors". Binding to receptors induces activation of an action both of an inhibitory and excitatory nature, owing to the dual conformation of the receptor; these effects are strongly correlated with clinical expression, generally with a mix of analgesia (useful, antinociceptive expression) and adverse effects (harmful, excitatory expression). The presence of adverse effects is now documented extensively in the literature, where the variables both of the symptoms and of their frequency are described; the presence of adverse effects is statistically significant whenever a patient uses a drug of the opioid category, just as the level of discontinuation of therapy is also statistically significant, in relation to the presence of adverse effects, especially if there are symptoms such as vomiting, dizziness, and sensory disorientation. The presence of adverse effects can be predicted, but displays an individual range both with respect to the type of symptom induced and with respect to the intensity with which it is manifested. The treatment indicated in the literature in the presence of adverse effects is connected with just three options: suspend administration of the drug, supplement the treatment regimen with a drug that can control the symptom, change the opioid molecule to equianalgesic doses (switching or rotation of opioids).

The following are the most common adverse effects:
- Drowsiness: this is an effect that is generally manifested in the initial phase of taking opiates, against which tolerance develops in the first 3-4 days. This effect may be accompanied by sensory confusion and dizziness. If drowsiness lasts for a long time and is excessive, this may be a sign of incorrect indication for the use of opioids since if the pain is not opioid-sensitive the non-analgesic pharmacological effects are accentuated.
- Urinary retention: urinary catheterization or the use of microdoses of naloxone may sometimes prove necessary. There is development of tolerance.
- Constipation: tolerance does not develop and the most common side-effect is often one of the most unpleasant. It is necessary to check that there is no intestinal occlusion. It is treated with faecal emollients, cathartics and hydration. Some works have reported improvements with the use of oral naloxone (Kreek M. J.).
- Nausea and vomiting: These effects are potentiated by vestibular stimuli. They occur in 5-15% of cases with rapid development of tolerance. The opiates produce these effects by a triple mechanism of action. They act directly on the zone of the bulbo-mesencephalic chemoceptors activating the vomiting centres: this component is controllable with haloperidol (0.5-1 mg/1-2 times/day) or prochlorperazine (5.10 mg/2-3 days) or corticosteroids. The action on the vestibular centres causes vomiting on walking: for this component
- drugs against dizziness and motion sickness such as transdermal scopolamine have some degree of efficacy
- there is reduced gastric motility that responds to metoclopramide (10 mg for 3 days). If there is obvious intolerance to morphine it can be replaced with methadone, which produces this effect to a smaller extent.
- pruritus: The mechanism of action that produces this symptom is not known. It seems to be mediated by the receptors and/or is antagonized by small doses of naloxone. An effective drug for reducing pruritus is hydroxyzine; topical anaesthetics, corticosteroids and antihistamines are less useful.

However, if excessive doses have been taken, a condition of overdose "complication" may develop, producing both an effect of sedation, with alteration of the sensory state that may reach coma, and an effect on respiration with a severe reduction of the breathing and secondary rate, bradypnoea and/or prolonged apnoea.

These complications, which represent a medical emergency, are treated using drugs of the opioid antagonist category; this includes the molecules naloxone and naltrexone; these drugs are normally administered intravenously, with all the risks and complications associated with this route of administration; excessive or incorrect administration of these products can cause the development of a withdrawal syndrome, which may be severe (something that may occur for example in persons who have used opioids for a long time or use them at high doses, for example in drug addiction or in users with oncologic pain) and can cause serious damage (for example cardiac tachyarrhythmias, pulmonary oedema, severe psychomotor agitation).

Patent application WO 00/62757 describes compositions for oral or nasal administration of opioid antagonists and, in particular, liquid solutions of naloxone are described in which the active principles are present at a concentration of 0.5 - 5% w/v.

Italian patent application MI2001A000907 reports the use of very low doses of naltrexone in patients being treated with opioids for attenuating their undesirable side-effects; the opioid and the opioid antagonist are administered simultaneously in formulations for the oral route.

It is clear, however, that it would be extremely beneficial to have formulations for administration of opioid antagonists at low concentrations by the nasal route.

### SUMMARY OF THE INVENTION

Pharmaceutical formulations are described for administration of liquid solutions of low-dose naltrexone by the nasal route.

### Detailed description of the invention

It was found, surprisingly, that it is possible to administer naltrexone in the form of liquid solution at low dose by the nasal route with excellent results both for attenuation of the undesirable side-effects due to the administration of opioids and in the case of excessive ingestion thereof.

Low dose according to the invention means a dose below between 0.005-0.02% w/v.

The liquid formulations for nasal administration according to the invention contain amounts of naltrexone (normally in the form of hydrochloride salt) between 0.001-0.005-0.02% (w/v).

The liquid solutions according to the invention are normally aqueous solutions or aqueous-alcoholic solutions in which the alcohol is preferably ethanol in an amount of approx. 5% (w/v). In addition, the solutions contain a buffer, the purpose of which is to maintain the pH at the value at which the opioid antagonist is in the form of a salt, for example as hydrochloride. The buffers can be selected from the following: citric acid/sodium citrate, citric acid/sodium hydroxide, dibasic sodium phosphate/citric acid, dibasic sodium phosphate/monobasic potassium phosphate, acetic acid/sodium acetate. The excipients used for the compositions of this type comprise: antimicrobial preservatives, agents that increase the tonicity and agents that increase the viscosity of the solution (viscosity improvers).

Among the antimicrobial preservatives, we may mention: benzalkonium chloride, methylparaben, propylparaben, sodium benzoate, benzoic acid, phenylethyl alcohol or mixtures thereof; preferably in amounts between 0.005-0.50% (w/v), preferably 0.005-0.30% (w/v), more preferably 0.01-0.1% (w/v).

Agents that increase tonicity are for example: sodium chloride, dextrose, lactose or mixtures thereof; preferably in amounts between 0.1-5.0% (w/v), preferably 0.1-2.0% 0(w/v), more preferably 0.1-0.9% (w/v).

The viscosity improvers can be selected from: hydroxypropyl methylcellulose (hypromellose), hydroxyethyl cellulose, hydroxypropyl cellulose, methylcellulose, microcrystalline cellulose, carboxymethylcellulose sodium, xanthan gum or mixtures thereof; preferably in amounts between: 0.01-2.0% (w/v), preferably 0.02-1.0% (w/v), more preferably 0.05-0.5% (w/v).

The formulations according to the invention are prepared following the standard techniques employed for preparing solutions for nasal application.

The following are dissolved in a given amount of water: the preservative, the salts for the buffer, the agent for increasing osmolality, and then the viscosity improver. When the solution obtained is clear, dissolve the active principle and make the solution up to the required volume with water.

Some examples of possible formulations according to the invention are given below.

### Example 1

To prepare 100 ml of solution, proceed as follows:
Add 1 ml of 2% w/v solution of benzalkonium chloride, 0.14 g of anhydrous citric acid, 0.37 g of sodium citrate dihydrate and 0.72 g of sodium chloride to 50 ml of water, with stirring. When the components have dissolved completely, add 0.1 g of hypromellose and continue stirring until a clear solution is obtained. Finally add 0.1 g of naltrexone hydrochloride until completely dissolved. Make up the volume of the solution obtained to 100 ml with water, and filter if necessary.

### Qualitative-quantitative composition

Hydroxypropyl methylcellulose 0.2%w/v
Benzalkonium chloride 0.02%w/v
Anhydrous citric acid 0.14%w/v
Sodium citrate dihydrate 0.37%w/v
Sodium chloride 0.72%w/v
Naltrexone hydrochloride 0.010%w/v

The solution obtained has the following physical characteristics:
pH = 4.8
Density = 1.007 g/ml
Osmolality = 283 mOsmol/kg;
Viscosity = 4.52 mPa*s.

Following the same procedure as in example 1, the following formulations are prepared:

### Example 2

### Qualitative-quantitative composition

Hydroxypropyl methylcellulose 0.2%w/v
Sodium propyl parahydroxybenzoate 0.02%w/v
Anhydrous citric acid 0.14%w/v
Sodium citrate dihydrate 0.37%w/v
Sodium chloride 0.72%w/v
Naltrexone hydrochloride 0.010%w/v

The solution obtained has the following characteristics:
pH = 4.9
Density = 1.007 g/ml;
Osmolality = 278 mOsmol/kg;
Viscosity = 4.15 mPa*s.

### Example 3

### Qualitative-quantitative composition

Methylcellulose 0.5%w/v
Benzalkonium chloride 0.02%w/v
Anhydrous citric acid 0.14%w/v
Sodium citrate dihydrate 0.37%w/v
Sodium chloride 0.72%w/v
Naltrexone hydrochloride 0.010%w/v

The solution obtained has the following characteristics:
pH = 4.8
Density = 1.008 g/ml
Osmolality = 292 mOsmol/kg;
Viscosity = 2.27 mPa*s.

The formulations according to the invention can be administered, for example, in the form of a spray using suitable applicators capable of nebulizing a defined amount of solution in the nasal cavities. The amount is normally between 50 and 100 µl and can optionally be repeated if required.

### Experimental design

An experimental protocol and the results obtained in a clinical study, in which naltrexone was administered to patients being treated with morphine, are presented below.

| Incidence of adverse effects from morphine in patients (total number 80) before treatment with *Naltrexone* | | | |
|---|---|---|---|
| Constipation | 70% | Dizziness | 13.3% |
| Nausea | 53.3% | Drowsiness | 30% |
| Vomiting | 53.3% | Pruritus | 30% |
| Inappetence | 46.7% | Asthenia | 43.3% |
| Urinary retention | 40% | | |
| Headache | 6.7% | | |

| Oncologic patients receiving therapy with morphine orally (average dose 40 mg +/- 10 mg) treated with *Naltrexone -* Dose every 12 hours: used 1/2 hour before morphine | | |
|---|---|---|
| Total Patients: 75 | | |
| ***Naltrexone* dose** | **Benefit (%)¹** | **Analgesia (%)²** |
| 2.5 mcg | 25% | 45% |
| 2.5 mcg | 90% | 60% |
| 0.125 mcg | 100% | 60% |
| 5 mcg | 40% | 55% |
| 1 mg | 90% | 100% |
| 1 mg | 65% | 90% |
| 1 mg | 40% | 100% |
| 1 mg | 70% | 65% |
| 1 mg | 65% | 60% |
| 1 mg | 100% | 70% |
| 5 mcg | 60% | 45% |
| 5 mcg | 60% | 55% |
| 5 mcg | 0% | 70% |
| 5 mcg | 70% | 100% |
| 5 mcg | 50% | 90% |
| 5 mcg | 45% | 60% |
| 5 mcg | 85% | 90% |
| 5 mcg | 45% | 65% |

| ***Naltrexone* dose** | **Benefit (%)¹** | **Analgesia (%)²** |
|---|---|---|
| 2.5 mcg | 100% | 0% |
| 1 mg | 100% | 100% |
| 2.5 mcg | 80% | 45% |
| 2.5 mcg | 80% | 45% |
| 1 mg | 65% | 65%** |
| 2.5 mcg | 70% | 55% |
| 2.5 mcg | 50% | 60%** |
| 2.5 mcg | 80% | 45% |
| 0.125 mcg | 65% | 45% |
| 1 mg | 90% | 100% |
| 2.5 mcg | 65% | 100% |
| 5 mcg | 55% | 60%** |
| 1 mg | 75% | 55% |
| 1 mg | 95% | 75%** |

| **Naltrexone dose** | **Benefit (%)1** | **Analgesia (%)2** |
|---|---|---|
| 2.5 mcg | 50% | 70% |
| 2.5 mcg | 40% | 60% |
| 0.125 mcg | 65% | 45% |
| 0.125 mcg | 40% | 55%** |
| 1 mg | 65% | 65%** |
| 5 mcg | 35% | 55% |
| 2.5 mcg | 50% | 60% |
| 2.5 mcg | 60% | 55% |
| 1 mg | 70% | 70%** |
| 2.5 mcg | 60% | 100% |
| 2.5 mcg | 65% | 40% |
| 5 mcg | 45% | 60%** |
| 1 mg | 65% | 85% |
| 5 mcg | 55% | 65%** |

| **Naltrexone dose** | **Benefit (%)1** | **Analgesia (%)2** |
|---|---|---|
| 2.5 mcg/day | 90% | 60% |
| 0.125 mcg/day | 100% | 60% |
| 5 mcg | 40% | 55% |
| 1 mg | 90% | 100% |
| 1 mg | 65% | 90% |
| 1 mg | 40% | 100% |
| 1 mg | 70% | 65% |
| 5 mcg | 65% | 60% |
| 1 mg | 100% | 70% |
| 5 mcg | 60% | 45% |
| 5 mcg | 60% | 55% |
| 1 mg | 0% | 70% |
| 1 mg | 70% | 100% |
| 1 mg | 55% | 65%** |
| 5 mcg | 50% | 90% |

| **Naltrexone dose** | **Benefit (%)1** | **Analgesia (%)2** |
|---|---|---|
| 5 mcg | 100% | 55%** |
| 5 mcg | 100% | 100% |
| 2.5 mcg | 80% | 45% |
| 2.5 mcg | 80% | 45% |
| 0.125 mcg | 65% | 65%** |
| 2.5 mcg | 55% | 45% |
| 1 mg | 50% | 60% |
| 1 mg | 80% | 45%** |
| 1 mg | 65% | 45% |
| 1 mg | 80% | 100% |
| 5 mcg | 45% | 100% |
| 5 mcg | 50% | 60% |
| 1 mg | 65% | 85% |
| 5 mcg | 85% | 70%** |

| Spine patients undergoing therapy with morphine by the spinal route (average dose 4.8 mg + /- 0.4 mg) treated with *Naltrexone-*Dose every 12 hours: used ½ hour before epidural load |
|---|
| **Total Patients: 26** |

| ***Naltrexone dose*** | **Benefit (%)¹** | **Analgesia (%)²** |
|---|---|---|
| 2.5 mcg | 100% | 0% |
| 2.5 mcg | 100% | 100% |
| 2.5 mcg | 80% | 45% |
| 2.5 mcg | 80% | 85% |
| 5 mcg | 65% | 35% |
| 5 mcg | 70% | 85% |
| 1 mg | 50% | 60% |
| 2.5 mcg | 80% | 55% |
| 1 mg | 65% | 65% |
| 2.5 mcg | 95% | 100% |
| 2.5 mcg | 60% | 100% |
| 5 mcg | 50% | 60% |
| 1 mcg | 100% | 45% |

| ***Naltrexone dose*** | **Benefit (%)¹** | **Analgesia (%)²** |
|---|---|---|
| 5 mcg | 30% | 15% |
| 5 mcg | 50% | 60% |
| 2.5 mcg | 45% | 45% |
| 2.5 mcg | 80% | 75% |
| 1 mcg | 65% | 65% |
| 5 mcg | 70% | 45% |
| 2.5 mcg | 45% | 60% |
| 2.5 mcg | 60% | 55% |
| 1 mg | 65% | 95% |
| 5 mcg | 95% | 100% |
| 2.5 mcg | 60% | 90% |
| 5 mcg | 50% | 60% |
| 1 mcg | 70% | 45% |

| | | |
|---|---|---|
| Legend: ¹: Percentage reduction of adverse effects. ²: Analgesic efficacy after administration of naltrexone ** Reduction of efficacy after naltrexone | | |

## Claims

1. Pharmaceutical formulations in the form of liquid solution for spray administration by the nasal route containing naltrexone in amounts between 0.005-0.02% w/v.

2. Formulations according to Claim 1, **characterized in that** said liquid solutions are aqueous or aqueous-alcoholic solutions, in which the alcohol is ethanol in an amount of approx. 5% w/v.

3. Formulations according to Claims 1-2, **characterized in that** said formulations also comprise a buffer selected from: citric acid/sodium citrate, citric acid/sodium hydroxide, dibasic sodium phosphate/citric acid, dibasic sodium phosphate/monobasic potassium phosphate, acetic acid/sodium acetate buffer.

4. Formulations according to Claim 3 additionally containing: antimicrobial preservatives, agents that increase the tonicity and agents that increase the viscosity of the solution.

5. Formulations according to Claim 4, **characterized in that** said antimicrobial preservatives are: benzalkonium chloride, methylparaben, propylparaben, sodium benzoate, benzoic acid, phenylethyl alcohol or mixtures thereof and are contained in amounts between 0.005-0.50% (w/v), preferably 0.005-0.30% (w/v), more preferably 0.01-0.1 % (w/v).

6. Formulations according to Claims 1-5having the following compositions:
a) Hydroxypropyl methylcellulose 0.2%w/v
Benzalkonium chloride 0.02%w/v
Anhydrous citric acid 0.14%w/v
Sodium citrate dihydrate 0.37%w/v
Sodium chloride 0.72% w/v
Naltrexone hydrochloride 0.010%w/v
b) Hydroxypropyl methylcellulose 0.2%w/v
Sodium propyl parahydroxybenzoate 0.02%w/v
Anhydrous citric acid 0.14%w/v
Sodium citrate dihydrate 0.37%w/v
Sodium chloride 0.72% w/v
Naltrexone hydrochloride 0.010%w/v
c) Methylcellulose 0.5%w/v
Benzalkonium chloride 0.02%w/v
Anhydrous citric acid 0.14%w/v
Sodium citrate dihydrate 0.37%w/v
Sodium chloride 0.72% w/v
Naltrexone hydrochloride 0.010%w/v

## Patentansprüche

1. Pharmazeutische Formulierungen in Form einer flüssigen Lösung zur Anwendung als Sprüh über die Nasenwege, wobei diese Formulierungen Naltrexon in Mengen zwischen 0,005 und 0,02 Gew.-% enthalten.

2. Formulierungen nach Anspruch 1, **dadurch gekennzeichnet, dass** die genannten flüssigen Lösungen wässrige oder alkoholhaltige wässrige Lösungen sind, in denen es sich beim Alkohol um Ethanol in einer Menge von annähernd 5 Gew.-% handelt.

3. Formulierungen nach den Ansprüchen 1 - 2, **dadurch gekennzeichnet, dass** die genannten Formulierungen auch einen Puffer enthalten, der unter folgenden Puffersubstanzen ausgewählt wird:
Zitronensäure/Natriumcitrat,
Zitronensäure/Natriumhydroxid,
Dinatriumphosphat/Zitronensäure,
Dinatriumphosphat/Kaliumhydrogenphosphat,
Zitronensäure/Natriumacetat.

4. Formulierungen nach Anspruch 3, welche zusätzlich enthalten: antimikrobielle Konservierungsstoffe, Agenzien, welche die tonisierende Wirkung erhöhen, und Agenzien, welche die Viskosität der Lösung erhöhen.

5. Formulierungen nach Anspruch 4, **dadurch gekennzeichnet, dass** es sich bei den genannten antimikrobiellen Konservierungsstoffe um die Substanzen Benzalkoniumchlorid, Methylparaben, Propylparaben, Natriumbenzoat, Benzoesäure, Phenylethylalkohol oder Gemischen daraus handelt, die in Mengen zwischen 0,005 - 0,50 Gew.-%, vorzugsweise 0,005 - 0,30 Gew.-%, stärker vorzuziehen 0,01 - 0,1 Gew.-% enthalten sind.

6. Formulierungen nach den Ansprüchen 1 - 5, welche die folgenden Zusammensetzungen aufweisen:
| | |
|---|---|
| a) Hydroxypropylmethylcellulose | 0,2 Gew.-% |
| Benzalkoniumchlorid | 0,02 Gew.-% |
| wasserfreie Zitronensäure | 0,14 Gew.-% |
| Natriumcitratdihydrat | 0,37 Gew.-% |
| Natriumchlorid | 0,72 Gew.-% |
| Naltrexonhydrochlorid | 0,010 Gew.-% |
| b) Hydroxypropylmethylcellulose | 0,2 Gew.-% |
| Natriumpropylparahydroxybenzoat | 0,02 Gew.-% |
| wasserfreie Zitronensäure | 0,14 Gew.-% |
| Natriumcitratdihydrat | 0,37 Gew.-% |
| Natriumchlorid | 0,72 Gew.-% |
| | |
|---|---|
| Naltrexonhydrochlorid | 0,010 Gew.-% |
| c) Methylcellulose | 0,5 Gew.-% |
| Benzalkoniumchlorid | 0,02 Gew.-% |
| wasserfreie Zitronensäure | 0,14 Gew.-% |
| Natriumcitratdihydrat | 0,37 Gew.-% |
| Natriumchlorid | 0,72 Gew.-% |
| Naltrexonhydrochlorid | 0,010 Gew.-% |

## Revendications

1. Formulations pharmaceutiques sous la forme de solution liquide pour une administration par pulvérisation par la voie nasale contenant de la naltrexone dans des quantités situées entre 0,005 et 0,02 % p/v.

2. Formulations selon la revendication 1, **caractérisées en ce que** lesdites solutions liquides sont des solutions aqueuses ou hydro-alcooliques, dans lesquelles l'alcool est l'éthanol dans une quantité d'approximativement 5 % p/v.

3. Formulations selon les revendications 1 à 2, **caractérisées en ce que** lesdites formulations comprennent également un tampon choisi parmi : un tampon acide citrique/citrate de sodium, acide citrique/hydroxyde de sodium, phosphate disodique/acide citrique, phosphate disodique/phosphate monopotassique, acide acétique/acétate de sodium.

4. Formulations selon la revendication 3 contenant en outre : des conservateurs antimicrobiens, des agents qui augmentent la tonicité et des agents qui augmentent la viscosité de la solution.

5. Formulations selon la revendication 4, **caractérisées en ce que** lesdits conservateurs antimicrobiens sont : le chlorure de benzalkonium, le parahydroxybenzoate de méthyle, le parahydroxybenzoate de propyle, le benzoate de sodium, l'acide benzoïque, l'alcool phényléthylique ou leurs mélanges et sont contenus dans des quantités situées entre 0,005 et 0,50 % (p/v), de préférence 0,005 à 0,30 % (p/v), de manière davantage préférée 0,01 à 0,1 % (p/v).

6. Formulations selon les revendications 1 à 5 ayant les compositions suivantes :
a) Hydroxypropylméthylcellulose 0,2 % p/v
chlorure de benzalkonium 0,02 % p/v
acide citrique anhydre 0,14 % p/v
citrate de sodium dihydraté 0,37 % p/v
chlorure de sodium 0,72 % p/v
chlorhydrate de naltrexone 0,010 % p/v
b) Hydroxypropylméthylcellulose 0,2 % p/v
parahydroxybenzoate de propyle sodique 0,02 % p/v
acide citrique anhydre 0,14 % p/v
citrate de sodium dihydraté 0,37 % p/v
chlorure de sodium 0,72 % p/v
chlorhydrate de naltrexone 0,010 % p/v
c) Méthylcellulose 0,5 % p/v
chlorure de benzalkonium 0,02 % p/v
acide citrique anhydre 0,14 % p/v
citrate de sodium dihydraté 0,37 % p/v
chlorure de sodium 0,72 % p/v
chlorhydrate de naltrexone 0,010 % p/v.
